# EUROPEAN PATENT APPLICATION

(11) **EP 4 202 944 A1**
(43) Date of publication of application: **28.06.2023**
(21) Application number: 21858561.0
(22) Date of filing: 17.08.2021
(51) Int. Cl.: G16H 15/00, G16H 50/20, G16H 10/20, G16H 80/00, G16H 40/20, G16H 50/70

(54) **PROGRAMMABLE ELECTRONIC MEDICAL RECORD SYSTEM**

(30) Priority: 18.08.2020 KR 20200102942
(71) Applicant: Seoul National University Hospital, Seoul 03080 (KR)
(72) Inventor: KIM, Joonghee, Seongnam-si Gyeonggi-do 13596 (KR)
(74) Representative: V.O.
(86) International application number: PCT/KR2021/010894
(87) International publication number: WO 2022/039474

(57) **Abstract**

Provided is a programmable electronic medical record system capable of helping medical staff to efficiently and effectively process medical examination information. The electronic medical record system is a programmable electronic medical record system comprising: a medical staff terminal comprising an input window enabling the inputting of a statement script expressed using objects and methods, and an output window for displaying a data processing result according to the statement script; and an electronic medical record server communicating with the medical staff terminal, receiving the statement script and carrying out data processing which is preset, and then transmitting the result thereof to the medical staff terminal.

## Description

### Technical Field

The present invention relates to an electronic medical record system, and more particularly to a programmable electronic medical record system for which it is possible to directly program various orders in a medical staff terminal.

### Background Art

An electronic medical record system refers to a medical information system in which when a doctor directly inputs to a computer all information about a patient's clinical practice, all this material may be processed into a database to generate new information as well. With the introduction of the electronic medical record system, it has been possible to significantly reduce the work of medical institutions as compared with the conventional manner in which patient records were collected on paper manually. In particular, a series of procedures for locating a patient's medical record, transferring the same to a medical doctor, and re-receiving and dispensing a prescription are handled using a computer network, and thus it is possible to reduce patient waiting time and eliminate the need to provide a separate medical record room.

Despite such an electronic medical record system, medical practice has become increasingly difficult in recent years. Due to the aging, the problems of patients become increasingly complex and various tests increase, and thus information that one doctor must handle is rapidly increasing. Existing electronic medical record systems adopt a limited statement input method such as a mouse click based on a graphical user interface (GUI), and it is difficult to accurately process complex information according to various medical conditions.

### Detailed Description of the Invention

### Technical Problem

The present invention is to provide a programmable electronic medical record system that may help medical staff efficiently and effectively process medical information.

The problems to be solved by the present invention are not limited to the problems mentioned above, and other problems not mentioned can be clearly understood by those skilled in the art from the following description.

### Technical Solution

To this end, an electronic medical record system according to an embodiment of the present invention is a programmable electronic medical record system and includes: a medical staff terminal including an input window enabling the inputting of a statement script expressed using objects and methods, and an output window for displaying a data processing result according to the statement script; and an electronic medical record server communicating with the medical staff terminal, receiving the statement script and carrying out predetermined data processing, and then transmitting the result thereof to the medical staff terminal. Here, the electronic medical record server includes: an objectification unit for designating medical-related information as an object; a command processing unit for processing data according to objects and methods of the statement script; an embedding vector unit for converting a unique number and storage information into an N-dimensional embedding vector for each object designated by the objectification unit, where the N is a natural number; a probability calculating unit for calculating a probabilistic association between the objects designated by the objectification unit; and a dependent command designation unit for defining a command dependent on each object in advance so as to limit a content and a range of a command applicable to each object designated by the objectification unit.

The command processing unit may include: a delayed enforcement unit that enforces a predetermined statement when a preceding procedure is completed based on the statement script; and a condition enforcement unit which enforces a predetermined statement if a specific condition is satisfied based on the statement script.

The probability calculation unit may extract patients with a condition similar to that of a target patient by utilizing a patient object obtained by objectifying patient information and an embedding vector defined in each patient object, and then calculate the probability regarding an object associated with the target patient from statistical information of the patients extracted; or may construct a probabilistic association between various objects defined for a predetermined patient population with a statistical model, and then calculate the probability regarding the object associated with the target patient.

The medical-related information may include patient information, medical staff information, order information, medication information, treatment information, test information, medical record information, symptom information, and diagnostic information.

Details of other embodiments are included in the specification and drawings.

### Effects of Invention

As described above, with the programmable electronic medical record system according to the present invention, it is possible to operate and control electronic medical records actively via programming outside of the conventional limited input method of electronic medical records, thereby making it possible to perform medical tasks more efficiently. For example, when a medical staff, such as a doctor or the like, inputs a statement script composed of one or more statements via an input window of a terminal, an electronic medical record server processes data according to an object, a method, or the like represented in the input statement script. The medical staff can thus respond quickly and accurately to complex medical situations.

### Brief Description of the Drawings

FIG. 1 is a view showing a schematic configuration of an electronic medical record system according to an embodiment of the present invention.
FIG. 2 is a view showing a user interface of a medical staff terminal of FIG. 1.
FIG. 3 is a view showing a schematic configuration of an electronic medical record server of FIG. 1.

### Mode for Carrying Out the Invention

The advantages and features of the present invention, and a method of achieving the advantages and features will become apparent by reference to the following detailed description, taken in conjunction with the accompanying drawings. It should be understood, however, that the present invention is not limited to the embodiments set forth below, but is to be embodied in a variety of different forms; these embodiments are provided so that this disclosure will be thorough and complete, and will fully convey the scope of the invention to those skilled in the art, and the present invention is defined only by the scope of the claims. Like reference numerals refer to like elements throughout.

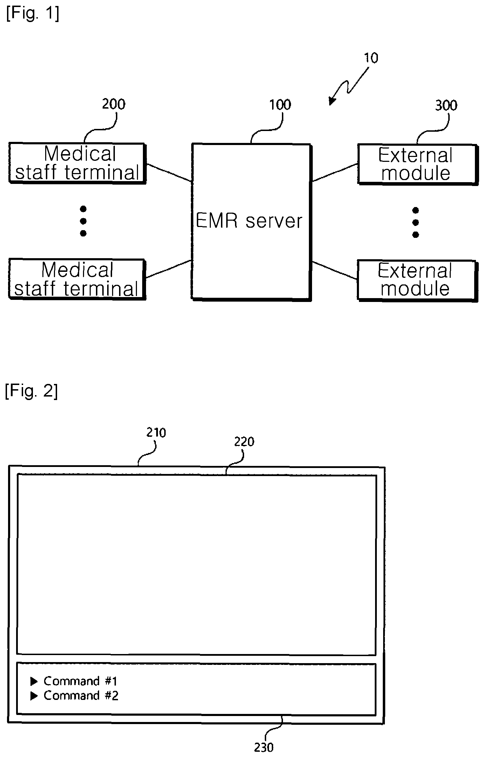

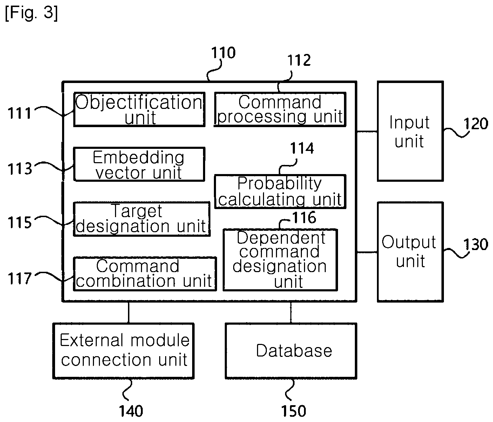

FIG. 1 is a view showing a schematic configuration of an electronic medical record system according to an embodiment of the present invention. FIG. 2 is a view showing a user interface of a medical staff terminal of FIG. 1. FIG. 3 is a view showing a schematic configuration of an electronic medical record server of FIG. 1.

An electronic medical record system 10 of the present invention, as a programmable system, may include an electronic medical record server 100, a medical staff terminal 200, an external module 300, and a wired/wireless network connecting the server and the terminal to each other.

The medical staff terminal 200 refers to a terminal capable of transmitting and receiving a variety of data to and from the electronic medical record server 100 via a wired or wireless network according to a key operation or command of a user. A user interface 210 of the medical staff terminal 200 includes an input window 230 enabling the inputting of a statement script to be transferred to the electronic medical record server 100, and an output window for displaying a data processing result according to the statement script. Here, the statement script may be expressed in an object-oriented programming language, for example, an object, a method, or the like. In object-oriented programming, a class defines attributes and behaviors belonging to the same group, and what is generated by the class and allocated to a memory during program execution is referred to as an instance or an object. A method is defined within a class and defines an operation in which an instance (or object) is performed in association with the class.

The medical staff terminal 200 may include a personal computer (PC), a tablet computer, a laptop computer, a PDA, or a mobile phone including a smartphone, and may also include a cloud computing terminal supporting cloud computing via a network.

The medical staff terminal 200 may communicate data with the electronic medical record server 100 by using a web browser, application software, or the like. Here, the application software may be application software downloaded by a user to the medical staff terminal 200 by using an external platform, or may be software installed in the medical staff terminal 200 by default in conjunction with operating system software.

The electronic medical record server 100 communicates with the medical staff terminal 200 and the external module 300, processes data according to a statement script transmitted from the medical staff terminal 200, and transmits a result therefrom to the medical staff terminal 200. The electronic medical record server 100 further includes storage means such as a database, means for managing, processing, and transmitting/receiving data, means for processing communication with an external device or an external system, and means for authentication and security, which may include various hardware tools, server devices, and pieces of software. Specifically, the electronic medical record server 100 includes a data processing unit 110, an input unit 120, an output unit 130, an external module connection unit 140, and a database 150.

The input unit 120 transfers information transmitted from the medical staff terminal 200, for example, a statement script, to the data processing unit 110, and the output unit 130 transmits a data processing result by the data processing unit 110 to the medical staff terminal 200.

The external module connection unit 140 is connected to an external module provided separately from the medical staff terminal 200 and transmits/receives a variety of data. For example, the external module may be implemented as a medical tool, a personalization tool, an external DB, an Al system, or the like.

The database stores all information for electronic medical records.

The data processing unit 110 includes: an objectification unit 111; a command processing unit 112; an embedding vector unit 113; a probability calculating unit 114; a target designation unit 115; a dependent command designation unit 116; and a command combination unit 117.

The objectification unit 111 designates all medical-related information as objects. For example, the objectification unit 11 designates and stores, as objects, medical-related information patient information, medical staff information, order information, medication information, treatment information, test information, medical record information, symptom information, diagnostic information, or the like. In addition, the objectification unit 111 may designate information about various external modules as objects, and a medical staff may call external module objects via the medical staff terminal 200 and directly control external modules via a statement script. The data processing unit 110 processes data in various ways on the basis of these objects.

The command processing unit 112 processes data according to an object and a method of a statement script input from the medical staff terminal 200. The command processing unit 112 may include a delayed enforcement unit and a condition enforcement unit.

The delayed enforcement unit enforces a predetermined statement upon completion of a preceding procedure on the basis of a statement script. Optionally, the delayed enforcement unit may provide predetermined notification information when a preceding procedure is completed. Usually, a medical task often proceeds sequentially, for example, a following procedure is performed after a specific test or treatment is completed in advance. Thus, when a statement script for delayed enforcement is input via the medical staff terminal 200 and a preceding procedure is thus completed, the delayed enforcement unit enforces a statement.

The condition enforcement unit enforces a predetermined statement when a specific condition is satisfied based on a statement script. The condition enforcement unit executes a statement after changing the contents of the statement in accordance with a condition described in a statement script. In actual medical practice, there are many cases in which decision-making changes depending on results of tests and treatments and patient progress; when a command to be performed for each condition is appropriately input via the medical staff terminal 200, the condition enforcement unit may perfectly implement a decision-making method in clinical practice by performing a corresponding command according to each condition.

The embedding vector unit 113 converts a unique number (key) and storage information (value) into an N-dimensional embedding vector for each object designated by the objectification unit 111 (here, N is a natural number). By this embedding vector, it is possible to effectively find an object with a unique number or storage information corresponding to a query given to use specific information.

The probability calculating unit 114 calculates a probabilistic association or a probability distribution between objects designated by the objectification unit 111 when a request is made from the medical staff terminal 200. As the probability calculating unit 114 may use patient information stored in the database 150, and population information or statistical information, a statistical model, or an artificial intelligence model provided in an external DB.

For example, the probability calculating unit 114 may use embedding vector information to extract patients in a state similar to that of a target patient, and calculate the probability regarding the target patient using statistical information obtained therefrom. Specifically, the probability calculating unit 114 may extract patients with a condition similar to that of a target patient by utilizing a patient object obtained by objectifying patient information and an embedding vector defined in each patient object, and then calculate the probability regarding an object associated with the target patient from statistical information of the extracted patients.

As another example, the probability calculating unit 114 may first construct a probabilistic association between various objects for a particular patient population into a statistical model, and then calculate the probability regarding a target patient using the statistical model. Specifically, the probability calculating unit 114 may construct a probabilistic association between various objects defined for a predetermined patient population into a statistical model, and then calculate the probability regarding an object associated with a target patient.

By using such a probability calculating unit 114, a large amount of electronical medical record information may be directly used clinically, and probability calculation by machine learning may be practically applied to clinical practice.

The target designation unit 115 designates a patient group to which a statement script is applicable. According to the statement script, the target designation unit 115 may designate all or a part of a patient object for which information inquiry or order application is required.

The dependent command designation unit 116 defines a command dependent on each object in advance so as to limit a content and a range of a command applicable to each object designated by the objectification unit 111. A medical staff may modify or add dependent commands as needed when inputting a statement script via the medical staff terminal 200. Therefore, it is possible to designate details suitable for each object for commands having the same name, and thus it is possible to simplify task processing.

The command combination unit 117 may specify and store a combination of statements as a single statement, and modify a content or add a function as needed. The command combination unit 117 may also be replaced with a statement having the same input and output as the command combination but having a different internal structure for application to an external module (for example, an artificial intelligence system or the like). When a medical staff inputs a statement script by the medical staff terminal 200, it is possible to recycle the command combination as needed to make the work efficient, and replace this command combination for practical application to an Al system.

The following embodiments are examples of a statement script input into the medical staff terminal 200. These examples illustrate a task processing procedure using an electronic medical record system according to an embodiment of the present invention.

### <Example 1>

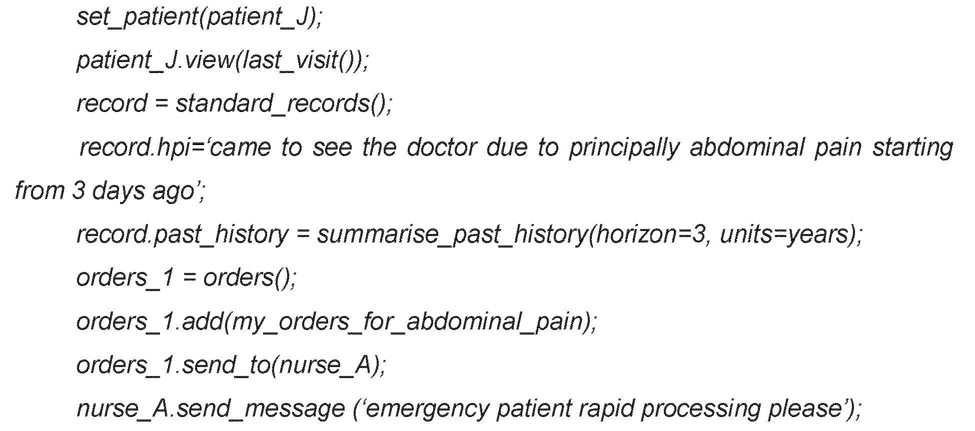

When a doctor inputs the statement script into the input window 230 of the medical staff terminal 200, the electronic medical record server 100 sequentially performs the followings. First, a patient object (patient_J) is generated for patient J, and the characteristics of the object are output. A record object is generated and a current medical history of patient J is input into an hpi sub-item. A past medical history within three years is collected by a predefined algorithm and input into a past_history item. An order object is generated and added to an abdominal pain order group (my_orders for abdominal_pain). An order and a message are transferred to a nurse object (nurse_A).

### <Example 2>

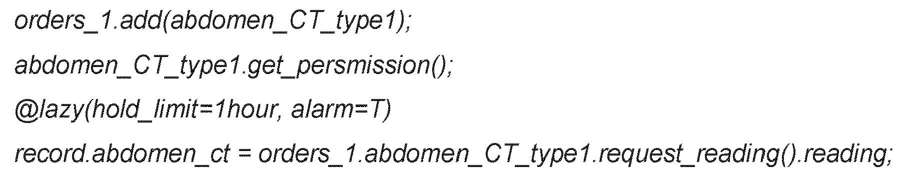

When a doctor inputs the statement script into the input window 230 of the medical staff terminal 200, the electronic medical record server 100 sequentially performs the followings. Abdominal CT is added to an order. Depending on added test attributes, some thereof are automatically ordered to an abdominal CT room. A command for receiving consent of abdominal CT is generated, and the command is transferred to a corresponding medical staff object as needed. When an abdominal CT test is completed, a readout operation is requested, and a readout result is input into medical records. By using delayed enforcement at this time, waiting is made until completion of a preceding procedure within a designated period of time, and in the case of exceeding the time, an alarm may be generated and transferred to a corresponding medical staff terminal.

### <Example 3>

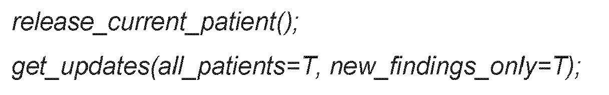

When a doctor inputs the statement script into the input window 230 of the medical staff terminal 200, the electronic medical record server 100 releases all currently selected patient objects and switches to an entire patient mode. New update information is then checked for all patients.

### <Example 4>

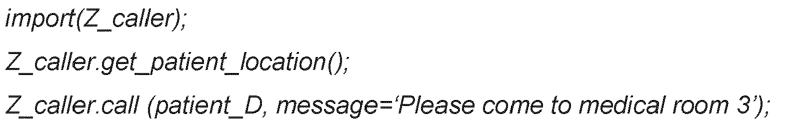

When a doctor inputs the statement script into the input window 230 of the medical staff terminal 200, the electronic medical record server 100 calls the external module 300, for example, a wireless remote wristband, to check the position of a patient. A specific message is transferred via the external module 300.

### <Example 5>

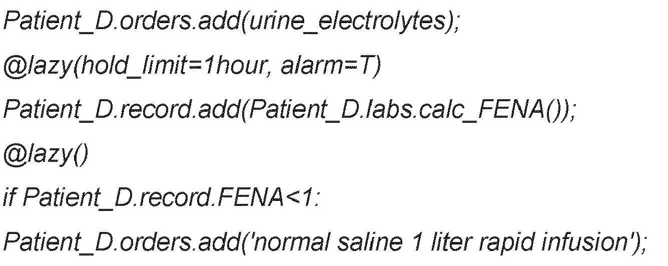

When a doctor inputs the statement script into the input window 230 of the medical staff terminal 200, the electronic medical record server 100 sequentially performs the followings. An order is added to a patient object (Patient_D). If a urinalysis occurs under delayed enforcement, FENA is calculated and input into medical records. If a specific condition (FENA<1) is satisfied in a conditional order format, an order is added.

### <Example 6>

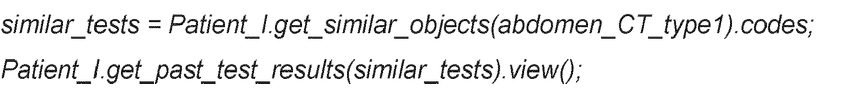

When a doctor inputs the statement script into the input window 230 of the medical staff terminal 200, the electronic medical record server 100 extracts codes of tests similar to an abdominal CT result object (abdomen_CT_type1) and outputs results of the similar tests. Since unique numbers and storage information for all test results have been converted into embedding vectors, similar tests may be extracted using vector similarity between the embedding vectors and results therefrom may be reviewed at once. That is, in the case of extracting a similarity test result with respect to a CT result of the abdomen, it is possible to extract similarity test results via vector similarity from various CT results such as a liver CT result, a pelvis CT result, a cecum CT result, a gynecological CT result, and an abdominal aorta CT result.

### <Example 7>

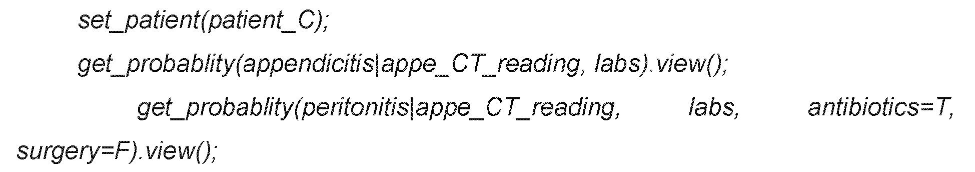

When a doctor inputs the statement script into the input window 230 of the medical staff terminal 200, the electronic medical record server 100 sequentially performs the followings. First, a patient object is generated for patient C. Based on a written CT readout for patient C, the probability of appendicitis is calculated. Based on a written CT readout for patient C, the probability that peritonitis occurs due to rupture of the cecum is calculated when observation is made for one week with only antibiotics without surgery in the current state.

While embodiments of the present invention have been described with reference to the accompanying drawings, it will be understood by those skilled in the art that the invention may be embodied in other specific forms without departing from the technical spirit or essential characteristics thereof. It is therefore to be understood that the embodiments described above are illustrative and not restrictive in all respects.

## Claims

1. A programmable electronic medical record system comprising:
a medical staff terminal comprising an input window enabling the inputting of a statement script expressed using objects and methods, and an output window for displaying a data processing result according to the statement script; and
an electronic medical record server communicating with the medical staff terminal, receiving the statement script and carrying out predetermined data processing, and then transmitting the result thereof to the medical staff terminal,
wherein the electronic medical record server comprises:
an objectification unit for designating medical-related information as an object; a command processing unit for processing data according to objects and methods of the statement script;
an embedding vector unit for converting a unique number and storage information into an N-dimensional embedding vector for each object designated by the objectification unit, where the N is a natural number;
a probability calculating unit for calculating a probabilistic association between the objects designated by the objectification unit; and
a dependent command designation unit for defining a command dependent on each object in advance so as to limit a content and a range of a command applicable to each object designated by the objectification unit.

2. The electronic medical record system according to claim 1, wherein the command processing unit comprises:
a delayed enforcement unit that enforces a predetermined statement when a preceding procedure is completed based on the statement script; and
a condition enforcement unit which enforces a predetermined statement if a specific condition is satisfied based on the statement script.

3. The electronic medical system according to claim 1, wherein the probability calculation unit extracts patients with a condition similar to that of a target patient by utilizing a patient object obtained by objectifying patient information and an embedding vector defined in each patient object, and then calculates the probability regarding an object associated with the target patient from statistical information of the patients extracted; or constructs a probabilistic association between various objects defined for a predetermined patient population with a statistical model, and then calculates the probability regarding the object associated with the target patient.

4. The electronic medical system according to claim 1, wherein the medical-related information includes patient information, medical staff information, order information, medication information, treatment information, test information, medical record information, symptom information, and diagnostic information.
